Europäisches Patentamt

European Patent Office

Office européen des brevets

(11) Veröffentlichungsnummer: **0 140 358**
**A2**

(12) **EUROPÄISCHE PATENTANMELDUNG**

(21) Anmeldenummer: **84112994.3**

(22) Anmeldetag: **27.10.84**

(51) Int. Cl.⁴: **A 01 M 1/20**
**A 01 M 13/00, A 61 L 9/12**

(30) Priorität: **01.11.83 DE 3339832**

(43) Veröffentlichungstag der Anmeldung:
**08.05.85 Patentblatt 85/19**

(84) Benannte Vertragsstaaten:
**AT BE CH DE FR GB IT LI**

(71) Anmelder: **Napierski, Reinhard**
**Talstrasse 18**
**D-6361 Niddatal 1(DE)**

(72) Erfinder: **Napierski, Reinhard**
**Talstrasse 18**
**D-6361 Niddatal 1(DE)**

(74) Vertreter: **Jochem, Bernd, Dipl.-Wirtsch.-Ing.**
**Patentanwälte Beyer & Jochem Postfach 17 01 45**
**D-6000 Frankfurt/Main(DE)**

(54) Elektrisches Gerät zum Verdampfen von Wirkstoffen, insbesondere Insektiziden.

(57) Das elektrische Gerät zum Verdampfen von in Trägerplättchen enthaltenen Wirkstoffen, insbesondere Insektiziden, besteht aus einem Gehäuse (10), das ein Magazin (22, 24, 32) zur Aufnahme mehrerer Trägerplättchen (18) enthält. Ein am Gehäuse beweglich gelagertes Transportorgan, wie z.B. ein drehbarer Käfig (32), transportiert durch seine Bewegung die gespeicherten Trägerplättchen aus der Speicherstellung im Magazin in den Raum zwischen Heizplatte (16) und Schutzgitter (26). Bei dem neuen Gerät braucht man nur in größeren Zeitabständen die Trägerplättchen mit den Fingern zu erfassen.

EP 0 140 358 A2

./...

Elektrisches Gerät zum Verdampfen von Wirk- **0140358**
stoffen, insbesondere Insektiziden

Die Erfindung betrifft ein elektrisches Gerät zum Verdampfen von in Trägerplättchen enthaltenen Wirkstoffen, insbesondere Insektiziden, bestehend aus einem mit einem Magazin für mehrere Trägerplättchen versehenen Gehäuse, welches eine durch ein Schutzgitter abgedeckte Heizplatte aufnimmt, wobei die Trägerplättchen in den Raum zwischen Heizplatte und Schutzgitter einführbar sind.

Es ist bekannt (DE-GM 78 23 826), Trägerplättchen außerhalb des Geräts im abgeschlossenen Zustand zu lagern und je nach Bedarf einzeln in das jeweils nur zur Aufnahme eines Plättchens bestimmte Gerät einzusetzen. Dabei muß das Trägerplättchen in die vorbestimmte Lage zwischen der Heizplatte und dem Schutzgitter gebracht werden. Weil dies allein unter Zuhilfenahme der Finger und loser Gegenstände, z.B. eines zweiten Trägerplättchens, nicht zwangsläufig zur lagerichtigen Positionierung der Trägerplättchen an der Heizplatte führt, sieht das bekannte Gerät einen am Gehäuse hin und her verschieblichen Rahmen mit einer Fensteröffnung vor, in die ein einzelnes Trägerplättchen außerhalb des Schutzgitters eingesetzt werden kann. Schiebt man dann den Rahmen in seine durch Anschlag genau bestimmte Endstellung unter das Schutzgitter, so kommt das Trägerplättchen exakt in die richtige Stellung an der Heizplatte. Somit erreicht man mit dem bekannten Schieberahmen nicht nur, wie beim einfachen Einlegen von Plättchen in einen zur Heizplatte führenden Schlitz, eine seitliche Führung der Plättchen, sondern im Laufe der seitlich geführten Relativbewegung zwischen Trägerplättchen und Heizplatte das Abstoppen dieser Bewegung durch Anschlag genau im richtigen Moment, so daß das Trägerplättchen gerade weit genug, aber nicht zu weit über die Heizplatte hinweggeschoben wird. Bei dem bekannten Gerät stellt demnach im Vergleich zum früher bekannten einfachen Stand der Technik mit Führungsschlitz der Schieberahmen ein Positionierhilfsmittel dar, welches das Hinwegschieben eines Trägerplättchens über die Heizplatte verhindert.

Es ist weiterhin bekannt (DE-GM 82 33 o77), im Gerät selbst einen abschließbaren Magazinbehälter vorzusehen, der eine Vielzahl von Trägerplättchen aufnehmen kann. Bei der bekannten Ausführung müssen diese Trägerplättchen jedoch von Hand aus dem Magazin entnommen und mit den Fingern in den Spalt zwischen Heizplatte und Schutzgitter eingeführt werden. Das Auswechseln der Trägerplättchen muß, da diese nur während verhältnismäßig kurzer Zeit wirksam sind, praktisch alle ein bis zwei Tage vorgenommen werden, wobei man bei allen vorbekannten Geräten bisher die vielfach giftige Substanzen enthaltenden Trägerplättchen jedesmal mit den Fingern anfassen muß. Außerdem ist bisher regelmäßig beim Auswechseln der Wirkstoffplättchen ein umständlicher Weg erforderlich, um das verbrauchte Plättchen in den nächstgelegenen Abfalleimer zu werfen.

Der Erfindung liegt die Aufgabe zugrunde, ein Gerät der eingangs genannten Art zu schaffen, welches den Gedanken der Magazinierung der Trägerplättchen im Gerät dahingehend weiter vervollkommnet, daß der gesamte Inhalt eines Magazins hintereinander verbraucht werden kann, ohne daß zwischendurch Trägerplättchen angefasst und beseitigt werden müssten.

Vorstehende Aufgabe wird erfindungsgemäß gelöst durch ein am Gehäuse beweglich gelagertes Transportorgan, durch dessen Bewegung die gespeicherten Trägerplättchen aus der Speicherstellung im Magazin in den Raum zwischen Heizplatte und Schutzgitter transportierbar sind.

Der Erfindung kommt es dabei auf die bequeme und überlange Zeit berührungsfreie Handhabung der Trägerplättchen an. Dieses Ziel ist wichtiger als die absolut genaue Positionierung der Trägerplättchen an der Heizplatte. In einfacher Ausführung der Erfindung genügt es deshalb, wenn das Transportorgan derart beweglich am Gehäuse gelagert ist, daß die mitgenommenen Trägerplättchen an der Heizplatte vorbeigeführt werden. Da man
das Trägerplättchen und die Heizplatte durch das Schutzgitter hindurch beobachten kann, lässt sich die Einstellung
mit Augenmaß vornehmen. Diese Genauigkeit genügt für die
praktische Anwendung. Selbstverständlich schließt dies nicht
aus, daß, falls gewünscht, zusätzliche Markierungen als optische Hilfsmittel, Rastungen als spürbare, aber nicht zwangsläufige Hilfsmittel oder auch Anschläge zur Positionierung
vorgesehen werden.

Wie die nachfolgende Beschreibung mehrerer Ausführungsbeispiele erfindungsgemäßer Geräte zeigt, kann das Transportorgan z.B. die Form einer relativ zum Gehäuse drehbaren
Trommel oder Scheibe oder die Form eines Schiebers haben. Je
nach Wahl der Beschickung des Magazins können weitere Bewegungsmöglichkeiten des Transportorgans allein zu Beschikkungszwecken vorgesehen sein, um den oder die Speicherplätze
im Magazin freizulegen. Alternativ besteht daneben selbstverständlich aber auch die Möglichkeit, mittels einer oder mehrerer verschließbarer Öffnungen am Gehäuse das Magazin zu füllen
und die verbrauchten Trägerplättchen zu entnehmen, ohne daß
hierzu das Transportorgan eine zu seiner eigentlichen Transportbewegung hinzukommende weitere Bewegung ausführen müsste.

In der Zeichnung zeigen:

Fig. 1
und 2     Längs- und Querschnitt eines Geräts mit Trommelmagazin;

Fig. 3
und 4     Draufsicht und Seitenansicht eines Geräts mit
          Magazin- und Abfallbehälter sowie einem schei-
          benförmigen, drehbaren Transportorgan zum Ein-
          zeltransport der Trägerplättchen vom Magazinbe-
          hälter über den Raum zwischen Heizung und Schutzgitter zum Abfallbehälter;

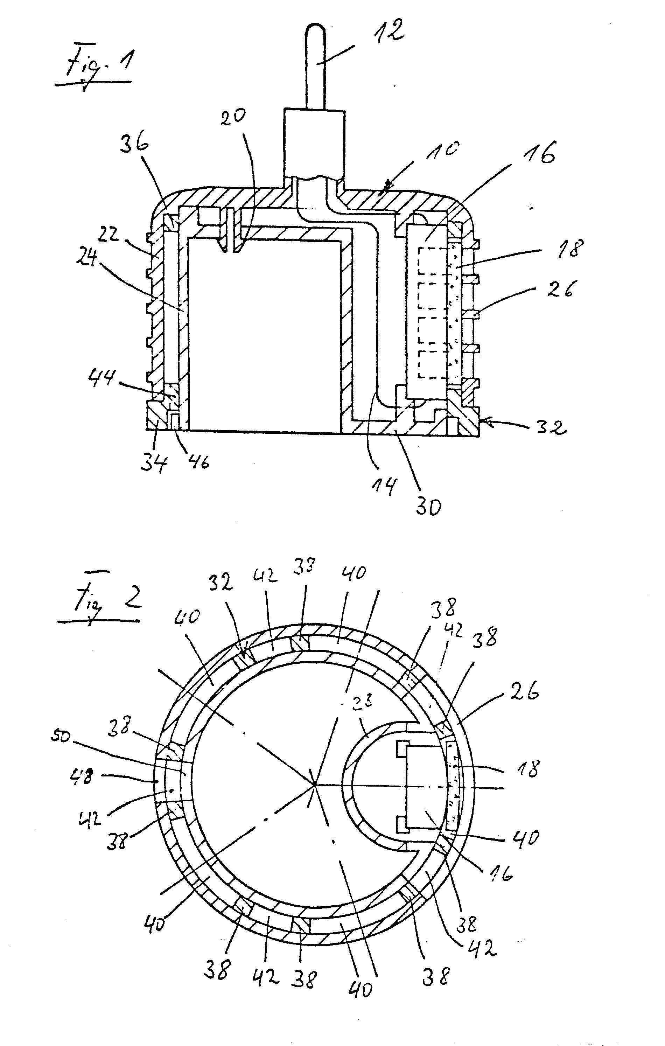

Fig. 5A,
B,C       Teilquerschnitte durch das Geräte nach Fig. 3
          und 4 zur Veranschaulichung des Wegs eines
          Trägerplättchens vom Magazin zum Abfallbehälter;

Fig. 6
und 7     Längsschnitte durch ein Gerät mit hin- und her-
          verschieblichem Transportorgan in zwei ver-
          schiedenen Stellungen;

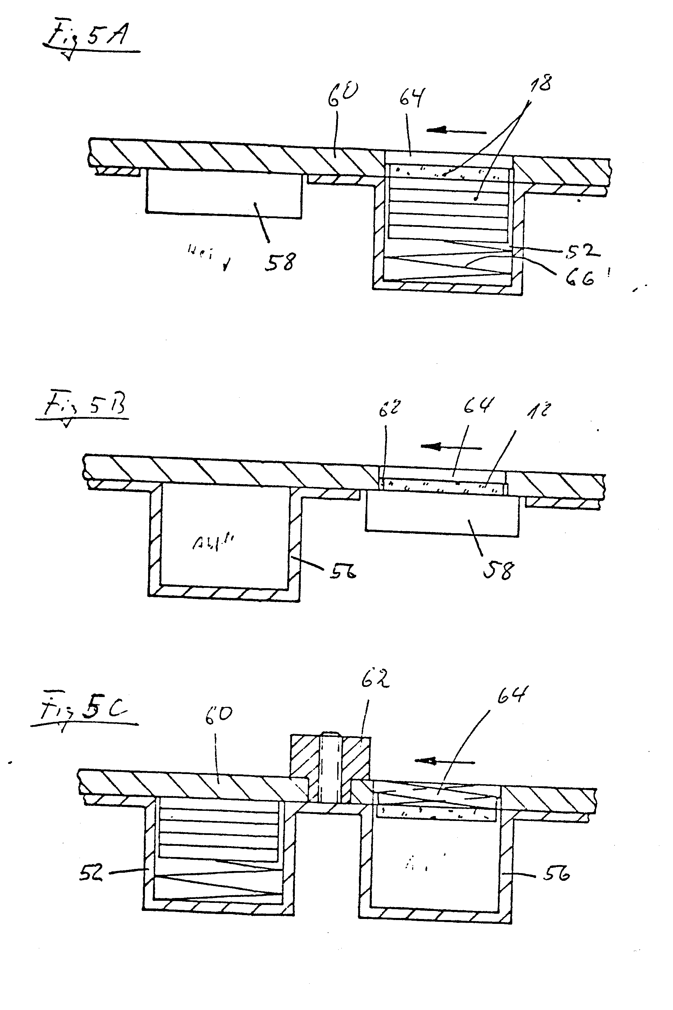

Fig. 8
und 9     Querschnitte durch das Gerät nach Fig. 6 und 7
          entsprechend den dort angegebenen Schnittlinien.

Das in Fig. 1 und 2 gezeigte Gerät besteht aus einem Gehäuse
1o mit elektrischen Steckerstiften 12 zum Einstecken in eine
übliche Steckdose. Die Stifte 12 sind in herkömmlicher Weise
über eine elektrische Leitung 14 mit einer grundsätzlich für
solche Zwecke bekannten Heizplatte 16 verbunden. Es könnte
zusätzlich eine Schalteinrichtung vorgesehen sein, um die Heizplatte ein- und auszuschalten oder auf verschiedene Heizstufen
umzuschalten. Letzteres käme z.B. dann in Frage, wenn Trägerplättchen 18 mit unterschiedlichen Wirkstoffen Verwendung
finden sollen.

Das Gehäuse 1o besteht im Ausführungsbeispiel aus zwei durch
eine Schnapp- oder Einrastverbindung 2o miteinander verbundenen Gehäuseteilen 22, 24, die eine äußere bzw. innere Mantelwand bilden. Während im Beispielsfall das innere Gehäuseteil 24 die Heizplatte 16 trägt, ist das äußere Gehäuseteil
22 an einer Stelle seiner Umfangswand als luftdurchlässiges Schutzgitter 26, d.h. als ein vergittertes offenes Fenster,
ausgebildet. Die Heizplatte 16 liegt in radialer Richtung
in Flucht mit dem Schutzgitter 26. Im übrigen ist die Heizplatte 16 durch eine Einwölbung 28 des inneren Gehäuseteils
24 radial nach innen abgedeckt. Ein frontseitiger Abschluß
3o dieser Einwölbung verhindert auch die Berührung der Heizplatte von vorn.

Im Ringraum zwischen den Mantelwänden der Gehäuseteile 22
und 24 ist ein Käfig 32 drehbar geführt. Dieser Käfig besteht aus einem vorderen Griffring 34, einem hinteren Ring
36 und sich zwischen den beiden Ringen in axialer Richtung
erstreckenden Stegen 38. Letztere bilden im Beispielsfall
fünf Taschen 4o zur Aufnahme jeweils eines Trägerplättchens
18. Jeweils zwischen zwei Taschen 4o hat der Käfig 32 eine
Durchbrechung 42, welche ähnlich wie eine Tasche ausgebildet,
jedoch schmaler ist. Diese Durchbrechungen 42 dienen als
Konvektionsöffnungen zur Durchlüftung des Geräts im eingeschalteten Zustand, wobei speziell die beiden Durchbrechungen neben der zwischen Heizplatte 16 und Schutzgitter 26
befindlichen Tasche 4o die Erwärmung der benachbarten Trägerplättchen, welche noch nicht oder nicht mehr an der Heizplatte 16 anliegen, verhindern.

Um das Trommelmagazin zu beladen, lässt sich der Käfig 32
teilweise axial nach vorn aus dem Gehäuse 1o herausziehen.
Zu diesem Zweck hat der vordere Ring 34 einen oder mehrere
in Fig. 1 bei 44 gestrichelt gezeigte innere Nuten, die
axial zur Flucht gebracht werden können mit einer entsprechenden Anzahl durch die Längsnuten 44 passender Anschläge 46
am Gehäuseteil 24. Vorzugsweise fluchten die Nuten 44 und
Anschläge 46 nur dann, wenn sich nicht gerade eine der Taschen
4o zwischen Heizplatte 16 und Schutzgitter 26 befindet. Dadurch wird verhindert, daß dann, wenn das Gerät in Betrieb
ist, Kinder in einfacher Weise den Käfig 32 axial herausziehen können. Die fluchtende Stellung von Nuten 44 und Anschlägen 46 kann durch geeignete äußere Markierungen am Gehäuse und am Ring 34 angezeigt werden.

Bei dem gezeigten Ausführungsbeispiel haben auch die Mantelwände des Gehäuses 1o fluchtende Durchbrechungen 48, 5o, die zu beiden Seiten der Heizplatte und dieser diametral gegenüberliegend mit den Durchbrechungen 42 im Käfig fluchten, wenn sich eine Tasche 4o zwischen Heizplatte 16 und Schutzgitter 26 befindet.

Es ist aus der Zeichnung ersichtlich, daß von den fünf Taschen 4o in der Normalstellung nach Fig. 2 jeweils 4 Taschen durch die innere und äußere Mantelwand des Gehäuses, die Stege 38 und vorderen Ring 34 des Käfigs dicht abgeschlossen sind, während die sich zwischen der Heizplatte 16 und dem Schutzgitter 26 befindende Tasche 4o zur Außenatmosphäre geöffnet ist, so daß die mittels der Heizplatte verdünsteten Wirkstoffe eines Trägerplättchens 18 in den Raum gelangen. Dagegen bleiben die Wirkstoffe in den in den abgeschlossenen Taschen 4o aufgenommenen Trägerplättchen erhalten, bis auch diese Trägerplättchen mittels des Griffrings 34 am Käfig 32 in die Stellung vor der Heizplatte 16 gebracht werden.

Damit bei Drehung des beladenen Trommelmagazins die Trägerplättchen 18 nicht an der Kante der Heizplatte 16 anstoßen, ist diese an der in Drehrichtung vorderen und hinteren Ecke abgerundet oder abgeschrägt. Außerdem kann eine elastisch nachgiebige Lagerung der Heizplatte 16 vorgesehen sein, um für zuverlässige Anlage der Trägerplättchen an der Heizplatte zu sorgen.

Um das Magazin des Geräts nach Fig. 1 und 2 zu beladen, wird der Käfig 32 nur so weit nach vorn aus dem Gehäuse herausgezogen, daß fünf Trägerplättchen von Hand in die nicht ganz frei liegenden Taschen 4o eingelegt werden können. Dabei werden die Trägerplättchen bereits teilweise in den Ringraum
zwischen den beiden Gehäuseteilen 22 und 24 eingeschoben und
fallen nicht herunter, wenn man das Gerät in der Hand dreht,
um alle Taschen 4o zu beladen. Anschließend wird der Käfig
32 wieder in seine innere Lage im Gehäuse axial zurückgeschoben, wobei in dieser durch die Nuten 44 und Anschläge
46 bestimmten Stellung keines der in den Taschen 4o sitzenden Trägerplättchen richtig an der Heizplatte 16 anliegt.
Es bereitet aber keine Schwierigkeiten, durch Drehung des
als Transportorgan dienenden Käfigs 32 mit Blick durch das
Schutzgitter 26 nach Augenmaß ein Trägerplättchen 18 genau
über die Heizplatte 16 zu drehen.

Wenn nach der üblichen Gebrauchsdauer ein Trägerplättchen
18 verbraucht, d.h. unwirksam geworden ist, genügt es, einfach nur durch Verdrehen des Rings 34 relativ zum Gehäuse 1o
ein in einer anderen Tasche 4o des Magazins enthaltenes frisches Trägerplättchen in die Wirkstellung vor der Heizung zu
drehen. Die verbrauchten Trägerplättchen bleiben solange im
Gerät, bis sämtliche Trägerplättchen verbraucht sind. Im Beispielsfall braucht während einer ganzen Woche kein Trägerplättchen mit den Fingern angefasst zu werden. Außerdem geht
das Weiterdrehen des Käfigs in die nächste Wirkstellung sehr
viel schneller als das Auswechseln eines einzelnen Plättchens.

Das gezeigte Gerät gestattet zahlreiche Abwandlungen und Erweiterungen. Bei geeigneter Ausbildung der elektrischen Verbindung zwischen Kontaktstiften 12 und Heizplatte 16 könnte
der Käfig 32 feststehen und das Gehäuse mit samt Heizplatte
16 und Schutzgitter 26 drehbar gelagert sein. Es besteht außerdem die theoretische Möglichkeit, eine radial nach außen
und stirnseitig abgedeckte Heizplatte 16 am äußeren Gehäuseteil und das Schutzgitter 26 in der Mantelwand des inneren
Gehäuseteils 24 anzuordnen. Weiterhin müssen auch nicht unbedingt die Trägerplättchen 18 im wesentlichen in der gekrümmten Ebene der Mantelwand eines Trommelspeichers liegen.
Der Speicher könnte stattdessen auch die Form einer drehbaren
Scheibe mit Taschen zur Aufnahme der Trägerplättchen haben,
wobei im letzteren Fall das Gehäuse so zu konstruieren ist, daß
die Heizplatte 16 und das Schutzgitter 26 in Flucht axial zu
beiden Seiten der drehbaren Scheibe anzuordnen sind. Auch die
übrigen vorstehend beschriebenen Eigenschaften des Geräts
nach Fig. 1 und 2 lassen sich auf ein derartig abgewandeltes
Gerät mit scheibenförmigem statt trommelfrömigem Magazin umsetzen. Dabei können in beiden Fällen auch noch zusätzliche
Mittel, z.B. elastische Glieder, an den Taschen vorhanden
sein, um die Trägerplättchen zu klemmen. Selbst dann kann bei
geeigneter Auslegung der Form der Taschen 40 und der Klemmung zwischen den Ringen 34, 36 und/oder Stegen 38 das Entleeren der verbrauchten Trägerplättchen so einfach wie bei
dem beschriebenen Ausführungsbeispiel gehalten werden, d.h.
man braucht nur über einem Abfalleimer den Käfig 32 in seine
äußerste Lage aus dem Gehäuse herauszuziehen und das Gerät
ein wenig zu schütteln. Außerdem läßt sich jederzeit mit den
Fingern nachhelfen, wenn ein Plättchen verklemmt ist.

Darüberhinaus besteht die Erweiterungsmöglichkeit, in dem zur
Verfügung stehenden leeren Innenraum des Geräts nach Fig. 1
und 2 eine motorische Antriebseinrichtung, z.B. einen elektrischen oder Federmotor, und eine Zeitschalteinrichtung
unterzubringen, die in vorgesehenen zeitlichen Abständen das
Magazin schrittweise weiterschaltet, bis alle Trägerplättchen
verbraucht sind.

Das Gerät nach Fig. 3 bis 5 hat zwar ebenfalls ein drehbares
Transportorgan, aber einen gemeinsamen Magazinbehälter 52 für
sämtliche zu speichernden Trägerplättchen 18. Daneben ist in
dem mit 54 bezeichneten Gehäuse ein dem Magazinbehälter 52 entsprechender Abfallbehälter 56 für verbrauchte Trägerplättchen
ausgebildet. Schließlich enthält das Gehäuse 54 in der aus
Fig. 3 ersichtlichen kreisförmigen Anordnung eine Heizplatte 58,
die in herkömmlicher Weise mit in das Gehäuse eingelassenen
Steckerstiften 12 verbunden ist. Das Transportorgan ist eine

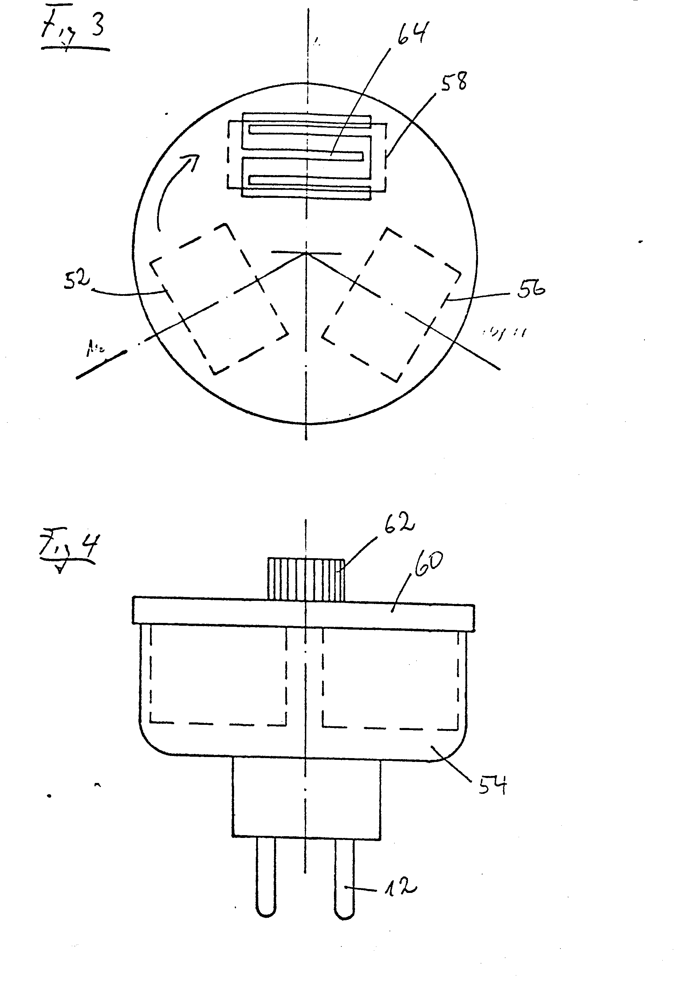

Scheibe 6o, die auf der Oberseite des Gehäuses 54 drehbar gelagert ist und z.B. mittels eines drehfest verbundenen Knopfs
62 gedreht werden kann. An einer Stelle ist die Scheibe 6o
durchbrochen und mit einem Schutzgitter 64 versehen. Wie aus
Fig. 5 ersichtlich, ist das Schutzgitter 64 schmaler als die
Dicke der Scheibe 6O, so daß jeweils ein Trägerplättchen 18
aus dem Magazinbehälter 52 unter der Wirkung einer Feder 66
bis zum Schutzgitter 64 in die Durchbrechung eintreten kann,
welche insofern eine in Fig. 5 mit 68 bezeichnete Tasche bildet, die nach außen durch das Schutzgitter 64 begrenzt ist.

Stellt man nun die Scheibe 6O so , daß sich die Tasche 68 gerade
über dem Magazinbehälter 52 befindet, so drückt die Feder 66
ein Trägerplättchen 18 in die Tasche 68 hinein, wie dies in
Fig. 5 A gezeigt ist. Anschließend kann man die Scheibe 6O
in der in Fig. 3 gezeigten Pfeilrichtung soweit drehen, bis
sich die Tasche 68 mit dem Schutzgitter 64 über der Heizplatte
58 befindet. In dieser Stellung kann mittels der Heizplatte 58
der Wirkstoff aus dem Trägerplättchen verdampft werden. Ist
dieses verbraucht, so wird die Scheibe 6O in Pfeilrichtung gemäß Fig. 3 und gemäß Fig. 5B weiter gedreht, bis die Tasche 68
das verbrauchte Trägerplättchen 18 von der Heizung 58 zum Abfallbehälter 56 transportiert hat. Dort fällt das verbrauchte
Trägerplättchen 18 infolge Schwerkraft in den Abfallbehälter 56.
Um den Abwurf sicherzustellen, wird vorzugsweise das Schutzgitter 64 gemäß Fig. 5C mit federnden Gitterstäben ausgebildet,
deren Federkraft schwächer ist als die Kraft der Feder 66, aber
ausreicht, um gemäß Fig. 5C das verbrauchte Trägerplättchen 18
aus der Tasche 68 in den Abfallbehälter 56 zu drücken. Bis zum
Abwurf des Trägerplättchens im Abfallbehälter 56 drückt das
elastische Schutzgitter 64 das in der Tasche 68 befindliche
Trägerplättchen gegen die obere Gleitfläche des Gehäuses bzw.
gegen die Heizplatte 58. Nach Verbrauch aller Trägerplättchen
kann die drehbare Scheibe 6O vom Gehäuse abgenommen, der Abfallbehälter 56 entleert und ein frischer Vorrat von Trägerplättchen in den Magazinbehälter 52 eingesetzt werden.

Es versteht sich, daß die im Zusammenhang mit Fig. 1 und 2 genannten ergänzenden Maßnahmen, wie z.B. Vorkehrungen zur Erzielung einer kühlenden Konvektion oder ein Zeitschaltmechanismus auch bei der Ausführung nach Fig. 3 bis 5 vorhanden sein können.

Das Ausführungsbeispiel nach Fig. 6 bis 9 zeigt ein Gerät mit einem Gehäuse 70 mit Kontaktstiften 12, einer Heizung 72, einem Magazinbehälter 74 und einem Abfallbehälter 76. Als Transportorgan dient ein hin- und hergehend beweglich am Gehäuse geführter Schieber 78, der in ähnlicher Weise wie die Scheibe 60 mit einer Durchbrechung mit Schutzgitter 80 (in Fig. 6 und 7 gestrichelt angedeutet) versehen ist. Unter dem Schutzgitter 80 befindet sich ebenfalls wieder analog zur Scheibe 60 eine Tasche 82, in die mittels einer Feder 84 ein Trägerplättchen 18 hineingedrückt werden kann, wenn sich die Tasche 82 über dem Magazinbehälter 74 befindet.

Im Ausführungsbeispiel liegt der Abfallbehälter 76 auf dem Verschiebeweg zwischen Magazinbehälter 74 und Heizung 72. Damit ein in der Tasche 82 mitgeführtes Trägerplättchen 18 auf dem Weg vom Magazinbehälter 74 zur Heizung 72 nicht in den Abfallbehälter 76 fällt, ist dieser durch eine elastische, klappenförmige Weiche 86 abgedeckt. Diese ist zu der in Fig.6 gezeigten Stellung hin elastisch vorgespannt, kann aber dann, wenn ein Trägerplättchen 18 in Richtung vom Magazinbehälter 74 zur Heizung 72 hinübergeführt wird, elastisch nachgiebig ausweichen. Wird dann jedoch der Schieber 78 aus der Stellung nach Fig. 6 in die nach Fig. 7 zurückgeschoben, ergreift die Weiche 86 in der in Fig. 7 gezeigten Weise das verbrauchte Trägerplättchen 18 und leitet es in den Abfallbehälter 76 (siehe gestrichelte Darstellung in Fig. 7). Die leere Tasche 82 kann dann in der in Fig. 7 in ausgezogene Linien gezeigten Stellung über dem Magazinbehälter 74 das nächste frische Trägerplättchen 18 aufnehmen.

Das die Tasche 82 seitlich begrenzende hakenförmige Ende des Schiebers 78 gelangt gemäß Fig. 7 an der Weiche 18 vorbei, weil beide Teile nicht über die gesamte Breite eines Trägerplättchens durchgehend ausgebildet sind, sondern versetzt zueinander bzw. auf Lücke angeordnet sind. Bei einer solchen
Ausbildung besteht auch die Möglichkeit, anders als in Fig. 7
gezeigt, die Heizung 72 durch ein fest am Gehäuse 70 angebrachtes Schutzgitter zu überdecken, welches sich mit der lückenhaften Vorderkante des Schiebers 78 verzahnt, wenn dieser in
die Stellung nach Fig. 6 verschoben wird.

Die Figuren 8 und 9 zeigen die Anordnung der vorstehend genannten Teile im Querschnitt, um sichtbar zu machen, wie bei
Längsbewegung des Schiebers 78 die Teile sich aneinander vorbei bewegen können.

Patentansprüche

1. Elektrisches Gerät zum Verdampfen von in Trägerplättchen enthaltenen Wirkstoffen, insbesondere Insektiziden, bestehend aus einem mit einem Magazin für mehrere Trägerplättchen versehenen Gehäuse, welches eine durch ein Schutzgitter abgedeckte Heizplatte aufnimmt, wobei die Trägerplättchen in den Raum zwischen Heizplatte und Schutzgitter einführbar sind, g e k e n n z e i c h n e t   d u r c h   ein am Gehäuse (10, 54, 70) beweglich gelagertes Transportorgan (32, 60, 78), durch dessen Bewegung die gespeicherten Trägerplättchen (18) aus der Speicherstellung im Magazin (40, 52, 74) in den Raum zwischen Heizplatte (16, 58, 72) und Schutzgitter (26, 74, 80) transportierbar sind.

2. Gerät nach Anspruch 1, d a d u r c h   g e k e n n - z e i c h n e t ,   daß das Magazin ein Trommelmagazin (32) ist, in welchem die Trägerplättchen (18) am Trommelumfang gespeichert gehalten sind, wobei das Trommelmagazin (32) und eine am Trommelumfang angeordnete Heizung (16) mit Schutzgitter (26) relativ zueinander drehbar sind.

3. Gerät nach Anspruch 2, d a d u r c h   g e k e n n - z e i c h n e t ,   daß das Trommelmagazin als ein im ringförmigen Zwischenraum eines doppelwandigen Gehäuses (10, 22, 24) drehbar geführter Käfig (32) mit Taschen (40) zur Aufnahme der Trägerplättchen (18) ausgebildet ist, das Schutzgitter (26) an der einen und die Heizplatte (16) an der anderen Wand (22 bzw. 24) des Gehäuses (10) angeordnet sind.

4. Gerät nach Anspruch 3, d a d u r c h   g e k e n n - z e i c h n e t ,   daß der Käfig (32) zwischen den Taschen (40) Durchbrechungen (42) aufweist, welche in solchen Drehwinkelstellungen des Käfigs, bei denen sich eine Tasche (40) zwischen Heizplatte (16) und Schutzgitter (26) befindet, mit Durchbrechungen (48, 50) in den Gehäusewänden (22, 24) fluchten.

5. Gerät nach einem der Ansprüche 1 bis 4, d a d u r c h g e k e n n z e i c h n e t , daß in solchen Drehwinkelstellungen, bei welchen sich eine Tasche (40) zwischen Heizplatte (16) und Schutzgitter (26) befindet, der Käfig (32) und die Gehäusewände (22, 24) die übrigen Taschen (40) allseitig dicht abschließen.

6. Gerät nach einem der Ansprüche 1 bis 5, d a d u r c h g e k e n n z e i c h n e t , daß der Käfig (32) wenigstens so weit axial aus dem Gehäuse (10) herausziehbar ist, daß Trägerplättchen (18) in die Taschen (40) einlegbar sind.

7. Gerät nach Anspruch 6, d a d u r c h g e k e n n z e i c h n e t , daß der Käfig (32) nur in solchen Drehwinkelstellungen aus dem Gehäuse (10) herausziehbar ist, in welchen sich nicht eine der Taschen (40) zwischen Heizplatte (16) und Schutzgitter (26) befindet.

8. Gerät nach Anspruch 1, d a d u r c h g e k e n n z e i c h n e t , daß das Magazin ein topfförmiger Behälter (52, 74) ist, in welchem die gespeicherten Trägerplättchen (18) flach aneinander anliegen und unter einem zum Transportorgan (60, 78) hin wirkenden Federdruck (66, 84) stehen.

9. Gerät nach Anspruch 8, d a d u r c h g e k e n n z e i c h n e t , daß das Transportorgan eine drehbar am Gehäuse (54) gelagerte Scheibe (60) ist.

10. Gerät nach Anspruch 8, d a d u r c h g e k e n n z e i c h n e t , daß das Transportorgan ein am Gehäuse (70) hin- und hergehend verschieblich geführter Schieber (78) ist.

0140358

11. Gerät nach Anspruch 8 oder 9, d a d u r c h    g e -
k e n n z e i c h n e t ,    daß das Transportorgan (60,
78) mit einer zwischen dem Magazin (52, 74) und der
Heizplatte (58, 72) verschieblichen inneren Tasche (68,
82) zur Aufnahme eines Trägerplättchen (18) ausgebildet
ist, welche über das Schutzgitter (64, 80) ständig mit
der Außenatmosphäre in Verbindung steht.

12. Gerät nach Anspruch 11, d a d u r c h    g e k e n n -
z e i c h n e t ,    daß die Tasche (68) vom Magazin (52),
an der Heizplatte (58) vorbei, zu einem topfförmigen Abfallbehälter (56) verschieblich ist, in welchem das in
der Tasche (68) mitgeführte Trägerplättchen (18) durch
Schwerkraft und/oder Federkraft (64) ablegbar ist.

13. Gerät nach Anspruch 11, d a d u r c h    g e k e n n -
z e i c h n e t ,    daß die Tasche (82) vom Magazin (74),
über eine Weiche (86) an einem topfförmigen Abfallbehälter (76) hinweg, zur Heizplatte (72) bewegbar ist und bei
Rückwärtsbewegung ein in der Tasche (82) mitgeführtes
Trägerplättchen (18) durch die Weiche (86) in den Abfallbehälter (76) leitbar ist.

Fig. 1

Fig. 2

0140358

1/4

*Fig 3*

64

58

52

56

*Fig 4*

62

60

54

12

3/4

0140358.

**Fig 5A**

60  64  18

Heiv  58

52
66

**Fig 5B**

62  64  12

Au

56

58

**Fig 5C**

62

60  64

52  56